(19) Europäisches Patentamt
European Patent Office
Office européen des brevets

(11) **EP 3 824 293 B1**

(12) **EUROPEAN PATENT SPECIFICATION**

(45) Date of publication and mention
of the grant of the patent:
**27.08.2025 Bulletin 2025/35**

(21) Application number: **19749599.7**

(22) Date of filing: **17.07.2019**

(51) International Patent Classification (IPC):
**G01N 33/68** (2006.01)

(52) Cooperative Patent Classification (CPC):
**G01N 33/6896;** G01N 2333/4709; G01N 2800/2814;
G01N 2800/2821

(86) International application number:
**PCT/EP2019/069257**

(87) International publication number:
**WO 2020/016304 (23.01.2020 Gazette 2020/04)**

(54) **METHODS OF IDENTIFYING AN INDIVIDUAL AS HAVING OR BEING AT RISK OF DEVELOPING AN AMYLOID-POSITIVE DEMENTIA BASED ON MARKER MOLECULES AND RELATED USES**

VERFAHREN ZUR IDENTIFIZIERUNG EINER PERSON MIT ODER MIT DER GEFAHR DES AUFTRETENS EINER AMYLOID-POSITIVEN DEMENZ AUF DER GRUNDLAGE VON MARKERMOLEKÜLEN UND VERWANDTE VERWENDUNGEN

PROCÉDÉS D'IDENTIFICATION D'UN INDIVIDU COMME AYANT OU ÉTANT SUSCEPTIBLE DE DÉVELOPPER UNE DÉMENCE À LIAISON AMYLOÏDE POSITIVE, SUR LA BASE DE MARQUEURS MOLÉCULAIRES, ET UTILISATIONS ASSOCIÉES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.07.2018 US 201816039376**

(43) Date of publication of application:
**26.05.2021 Bulletin 2021/21**

(73) Proprietors:
- **GENENTECH, INC.**
  **South San Francisco, CA 94080 (US)**
- **Roche Diagnostics GmbH**
  **68305 Mannheim (DE)**
  Designated Contracting States:
  **DE**
- **F. Hoffmann-La Roche AG**
  **4070 Basel (CH)**
  Designated Contracting States:
  **AL AT BE BG CH CY CZ DK EE ES FI FR GB GR HR HU IE IS IT LI LT LU LV MC MK MT NL NO PL PT RO RS SE SI SK SM TR**

(72) Inventors:
- **BITTNER, Tobias**
  **82377 Penzberg (DE)**
- **KARL, Johann**
  **82377 Penzberg (DE)**
- **LIFKE, Valeria**
  **82377 Penzberg (DE)**
- **STEFFEN, Verena**
  **South San Francisco, California 94080-4990 (US)**
- **JOCHAM, Martina**
  **82377 Penzberg (DE)**

(74) Representative: **Simmons & Simmons LLP (Munich) et al**
**Lehel Carré**
**Gewürzmühlstraße 11**
**80538 Munich (DE)**

(56) References cited:
- **NI-CHUNG LEE ET AL: "Blood Beta-Amyloid and Tau in Down Syndrome: A Comparison with Alzheimer's Disease", FRONTIERS IN AGING NEUROSCIENCE, vol. 8, 17 January 2017 (2017-01-17), XP055485695, DOI: 10.3389/ fnagi.2016.00316**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- LIH-FEN LUE ET AL: "Amyloid Beta and Tau as Alzheimer's Disease Blood Biomarkers: Promise From New Technologies", NEUROLOGY AND THERAPY, vol. 6, no. S1, 1 July 2017 (2017-07-01), pages 25 - 36, XP055619117, ISSN: 2193-8253, DOI: 10.1007/s40120-017-0074-8
- AKINORI NAKAMURA ET AL: "High performance plasma amyloid-[beta] biomarkers for Alzheimer's disease", NATURE, vol. 554, no. 7691, 31 January 2018 (2018-01-31), London, pages 249 - 254, XP055618943, ISSN: 0028-0836, DOI: 10.1038/nature25456
- KEERTHANAA BALASUBRAMANIAN SHANTHI ET AL: "A systematic review and meta-analysis of plasma amyloid 1-42 and tau as biomarkers for Alzheimer's disease", SAGE OPEN MEDICINE, vol. 3, 1 January 2015 (2015-01-01), XP055624175, ISSN: 2050-3121, DOI: 10.1177/2050312115598250
- LIH-FEN LUE ET AL: "Plasma Levels of A[beta]42 and Tau Identified Probable Alzheimer's Dementia: Findings in Two Cohorts", FRONTIERS IN AGING NEUROSCIENCE, vol. 9, 24 July 2017 (2017-07-24), pages 226, XP055624191, DOI: 10.3389/fnagi.2017.00226
- YACHEN SHI ET AL: "Potential Value of Plasma Amyloid-[beta], Total Tau, and Neurofilament Light for Identification of Early Alzheimer's Disease", ACS CHEMICAL NEUROSCIENCE, vol. 10, no. 8, 13 June 2019 (2019-06-13), US, pages 3479 - 3485, XP055624257, ISSN: 1948-7193, DOI: 10.1021/acschemneuro.9b00095

**Description**

<u>Summary of the Invention:</u>

The present invention relates to identifying an individual as having or being at risk of developing an amyloid-positive dementia based on marker molecules amyloid β40 (Aβ40), amyloid β42 (Aβ42) and total tau (tTau) and the use of the marker molecules for the identification of an individual having or being at risk of developing an amyloid-positive dementia.

<u>Background of the Invention:</u>

Alzheimer's disease (AD) is a chronic neurodegenerative disease that usually starts slowly and worsens over time. It is the cause of 60-70% of cases of dementia. The most common early symptom is difficulty in remembering recent events (short-term memory loss). As the disease advances, symptoms can include problems with language, disorientation (including easily getting lost), mood swings, loss of motivation, not managing self care, and behavioral issues. As a person's condition declines, they often withdraw from family and society. Gradually, bodily functions are lost, ultimately leading to death. Although the speed of progression can vary, the typical life expectancy following diagnosis is three to nine years.

Alzheimer's disease is usually diagnosed based on the person's medical history, history from relatives, and behavioral observations. The presence of characteristic neurological and neuropsychological features and the absence of alternative conditions are supportive. Advanced medical imaging with computed tomography (CT) or magnetic resonance imaging (MRI), and with single-photon emission computed tomography (SPECT) or positron emission tomography (PET) can be used to help exclude other cerebral pathology or subtypes of dementia. Moreover, it may predict conversion from prodromal stages (mild cognitive impairment) to Alzheimer's disease. Assessment of intellectual functioning including memory testing can further characterize the state of the disease. Medical organizations have created diagnostic criteria to ease and standardize the diagnostic process for practicing physicians. The diagnosis can be confirmed with very high accuracy post-mortem when brain material is available and can be examined histologically.

The earliest pathological signature of Alzheimer's disease is deposition of the amyloid β proteins such as Aβ40 and Aβ42 in the brain and the only validated methods for identifying the same are amyloid β positron-emission tomography (PET) imaging or measurement of amyloid β in cerebrospinal fluid.

Also blood testing has been suggested in the art in order to identify amyloid-positive patients. Measurement of high-performance plasma amyloid β biomarkers by immunoprecipitation coupled with mass spectrometry has been described, wherein amyloid β precursor protein $(APP)_{669-711}$/Aβ42 and Aβ40/Aβ42 ratios were used to predict individual brain amyloid β-positive or amyloid β-negative status of a patient (Nakamura et al., 2018, Nature 554: 249-254).

However, the in vitro tests presently suggested do not meet the standards set by the applicant. Particularly, the intended high sensitivity (proportion of actual positives that are correctly identified as such) and high specificity (proportion of actual negatives that are correctly identified as such) are not reached.

Accordingly, there is still a need for a suitable method for identifying an individual as having or being at risk of developing an amyloid-positive dementia. Preferably, the methods should be inexpensive, robust and easy.

<u>Detailed Description of the Invention:</u>

Surprisingly, the inventors found the use of a combination of the marker molecules amyloid β40 (Aβ40), amyloid β42 (Aβ42) and total Tau (tTau) increased the quality of the analysis. This is particularly true, if a weighted calculation of the amount or concentration of the marker molecules determined in an individual's blood sample is used. Different populations were used in order to determine weighting factors for regression models. It could be shown that two models of the combination of the above markers Aβ40, Aβ42 and tTau (v = a * [Aβ40] + b * [Aβ42] + c * [tTau] + d (model 1) and v = e * [Aβ42] / [Aβ40] + f * [tTau] + g (model 2)) were superior to the established standard ([Aβ42] / [Aβ40]) (see Summary Table 5).

Accordingly, in a first aspect the present invention relates to a method of identifying an individual as having or being at risk of developing an amyloid-positive dementia, the method comprising

a) measuring in a sample obtained from the individual the amount or concentration of the marker molecules amyloid β40 (Aβ40), amyloid β42 (Aβ42) and total Tau (tTau), and
b) identifying the individual as having or being at risk of developing an amyloid-positive dementia by comparing the combined value for the markers determined in step (a) to a control value, wherein an increased combined value relative to the control value is indicative of the amyloid-positive dementia,

wherein the sample is not a brain sample or a cerebrospinal fluid sample, but a blood sample.

Dementia is a general term for loss of memory and other mental abilities severe enough to interfere with daily life. It is caused by physical changes in the brain. There are different types of dementia, such as Alzheimer's disease, vascular dementia, Lewy body dementia or fronto-temporal lobe dementia. Dementias can be divided into two groups, amyloid-positive and amyloid-negative dementias. Amyloid-positive dementias are characterized by amyloid plaques in the brain, wherein Alzheimer's disease is the most prominent form.

Alzheimer's disease has been identified as a protein misfolding disease (proteopathy), caused by plaques of abnormally folded amyloid β protein and tangles of tau protein in the brain. Plaques are made up of small peptides, 39 - 43 amino acids in length, called amyloid β peptides (Aβ). Aβ refers to a group of fragments from the larger amyloid β precursor protein (APP), a transmembrane protein that penetrates through the neuron's membrane. APP is critical to neuron growth, survival, and post-injury repair. In Alzheimer's disease, gamma secretase and beta secretase act together in a proteolytic process which causes APP to be divided into smaller fragments. One of these fragments gives rise to fibrils of amyloid β, which then form clumps that deposit outside neurons in dense formations known as senile plaques. Tangles are caused by abnormal aggregation of the tau protein, which usually stabilizes microtubules of the cytoskeleton when phosphorylated. In Alzheimer's disease, Tau becomes hyperphosphorylated and begins to pair with other threads, creating neurofibrillary tangles and disintegrating the neuron's transport system.

Exactly how disturbances of production and aggregation of the amyloid β peptide give rise to the pathology of Alzheimer's disease is not known. The amyloid hypothesis traditionally points to the accumulation of amyloid β peptides as the central event triggering neuron degeneration. Accumulation of aggregated amyloid fibrils, which are believed to be the toxic form of the protein responsible for disrupting the cell's calcium ion homeostasis, induces programmed cell death (apoptosis). It is also known that Aβ selectively builds up in the mitochondria in the cells of Alzheimer's-affected brains, and it also inhibits certain enzyme functions and the utilization of glucose by neurons.

The aim of the present invention is provide a method for reliably identifying an individual as having or being at risk of developing an amyloid-positive dementia. Accordingly, the individual may already show signs and symptoms of dementia and the method may be used in order to identify the present dementia as amyloid-positive or amyloid-negative. Alternatively, the individual may not yet show signs and symptoms of dementia. In this case the method may be used to identify the individual as being at risk of developing an amyloid-positive dementia.

In order to identify an individual as having or being at risk of developing an amyloid-positive dementia such as Alzheimer's disease or mixed dementia, a sample from the individual, is tested, wherein the sample is not a brain sample or a cerebrospinal fluid sample, but a blood sample. The individual according to the present invention may be any human or non-human animal, particularly a mammal, especially a human.

The sample may be any blood sample suitable for measuring the markers according to the present invention and refers to a biological sample obtained for the purpose of evaluation in vitro. It comprises material which can be specifically related to the individual and from which specific information about the individual can be determined, calculated or inferred. A sample can be composed in whole or in part of blood material from the individual. Preferably, the sample is easily accessible, to allow for inexpensive, robust and easy testing. According to the invention test samples include blood, serum, and plasma. The sample may be fluid or solid, e.g. a blood sample may be fluidic or may be a dried blood spot. The sample may be used immediately or processed before the measuring step a). Processing may include purification (e.g. separation such as centrifugation), concentration, dilution, elution (e.g. from solid carrier material), lysis of cellular components, freezing, acidification, conservation etc. Samples according to the invention include whole blood, serum, or plasma, with plasma representing the most preferred type of sample.

As a first step in the method of the invention, the amount or concentration of the marker molecules amyloid β40 (Aβ40), amyloid β42 (Aβ42) and total Tau (tTau) is measured in the sample.

The two most abundant alloforms of amyloid β peptide (found in amyloid deposits in the brain are 40 and 42 amino acids long (designated Aβ40 and Aβ42, respectively). Despite the small structural difference between these two peptides, they display distinct clinical, biological, and biophysical behavior.

Human Aβ40 and Aβ42 consist of amino acids 672-711 and 672-713, respectively, of protein APP (see database UniProtKB accession number: P05067). The statistically significant decrease of Aβ42 concentrations in the cerebrospinal fluid (CSF) of AD patients was confirmed in several studies. A decrease of the CSF Aβ42 has also been observed in patients with mild cognitive impairment (MCI). Several studies have shown that there is no change in the CSF level of Aβ40 in AD. Others have reported that a value of CSF Aβ ratio (Aβ40/Aβ42) provides a better discrimination between AD patients and controls or other dementias. The two proteins have also been suggested as candidate biomarkers in plasma samples (Okereke et al., 2009, J Alzheimers Dis 16(2): 277-285). Usually, the ratio of the proteins is used in the characterization of Alzheimer (Spies et al, 2010, Curr Alzheimer Res 7(5): 470-476) (Janelidze et al., 2016, Scientific Reports 6. Article number: 26801; doi:10.1038/srep26801).

Tau proteins are proteins that stabilize microtubules. They are abundant in neurons of the central nervous system and are less common elsewhere, but are also expressed at very low levels in CNS astrocytes and oligodendrocytes. Pathologies and dementias of the nervous system such as Alzheimer's disease and Parkinson's disease are associated with tau proteins that have become defective and no longer stabilize microtubules properly. Tau proteins are the products of alternative splicing from a single gene that in humans is designated MAPT (microtubule-associated protein Tau) and is located on chromosome 17. Sofar, six tau isoforms have been identified in human brain tissue, and they are distinguished by their number of binding domains. Details on the Tau isoforms are given in Hampel et al, 2010, Exp Gerontol 45: 30-44. Three isoforms have three binding domains and the other three have four binding domains. The isoforms with four binding domains are better at stabilizing microtubules than those with three binding domains. The isoforms are a result of alternative splicing in exons 2, 3, and 10 of the *tau* gene. Tau is a phosphoprotein with 79 potential Serine (Ser) and Threonine (Thr) phosphorylation sites on the longest Tau isoform. Phosphorylation has been reported on approximately 30 of these sites in normal Tau proteins. The degree of phosphorylation in all six isoforms decreases with age due to the activation of phosphatases. Hyperphosphorylation of the Tau protein (Tau inclusions, pTau) can result in the self-assembly of tangles of paired helical filaments and straight filaments, which are involved in the pathogenesis of Alzheimer's disease, frontotemporal dementia, and other tauopathies. All of the six tau isoforms are present in an often hyperphosphorylated state in paired helical filaments from Alzheimer's disease brain. When misfolded, this otherwise very soluble protein can form extremely insoluble aggregates that contribute to a number of neurodegenerative diseases. Tau proteins have a direct effect on the breakdown of a living cell caused by tangles that form and block nerve synapses. Tangles are clumps of Tau proteins that stick together and block essential nutrients that need to be distributed to cells in the brain, causing the cells to die. Accordingly, total Tau (tTau), which refers to all phosphorylated and non-phosphorylated isoforms of tau, and hyperphosphorylated tau (p-Tau) as well as A$\beta$42 in cerebrospinal fluid have been recommended as biomarkers in the diagnosis of Alzheimer, including its predementia and preclinical stages (Jack et al., 2011, Alzheimer's & Dementia 7: 257-262).

In accordance with the present invention, the amount or concentration of the markers is determined in order to identify an individual having or being at risk of developing an amyloid-positive dementia. The amount of a substance is a standards-defined quantity that measures the size of an ensemble of elementary entities, such as atoms, molecules, electrons, and other particles. It is sometimes referred to as chemical amount. The International System of Units (SI) defines the amount of substance to be proportional to the number of elementary entities present. The SI unit for amount of substance is the mole. It has the unit symbol mol. The concentration of a substance is the amount of a constituent divided by the total volume of a mixture. Several types of mathematical description can be distinguished: mass concentration, molar concentration, number concentration, and volume concentration. The term concentration can be applied to any kind of chemical mixture, but most frequently it refers to solutes and solvents in solutions. The molar (amount) concentration has variants such as normal concentration and osmotic concentration.

A variety of methods for measuring a marker molecule (particularly A$\beta$40, A$\beta$42 and tTau) are known in the art and any of these can be used. Exemplary methods are described in the Examples. Preferably, the marker(s) is/are specifically measured from a liquid sample by use of a specific binding agent.

A specific binding agent is, e.g., an antibody to the marker or a nucleic acid complementary to the nucleic acid relating to the marker protein (e.g. a nucleic acid complementary to a marker's mRNA or relevant part thereof). Preferably, the marker molecule(s) is/are measured at the protein level.

Determination of proteins as binding partners of a marker polypeptide can be performed using any of a number of known methods for identifying and obtaining proteins that specifically interact with proteins or polypeptides, for example, a yeast two-hybrid screening system such as that described in U.S. Pat. No. 5,283,173 and U.S. Pat. No. 5,468,614, or the equivalent. A specific binding agent has preferably at least an affinity of $10^7$ l/mol for its corresponding target molecule. The specific binding agent preferably has an affinity of $10^8$ l/mol or even more preferred of $10^9$ l/mol for its target molecule. As the skilled artisan will appreciate the term specific is used to indicate that other biomolecules present in the sample do not significantly bind to the binding agent specific for the marker. Preferably, the level of binding to a biomolecule other than the target molecule results in a binding affinity which is only 10% or less, more preferably only 5% or less of the affinity to the target molecule, respectively. A preferred specific binding agent will fulfill both the above minimum criteria for affinity as well as for specificity.

A specific binding agent preferably is an antibody reactive with any of the markers A$\beta$40, A$\beta$42 or tTau. The term antibody refers to a polyclonal antibody, a monoclonal antibody, antigen binding fragments of such antibodies, single chain antibodies as well as to genetic constructs comprising the binding domain of an antibody.

The term "antibodies" includes polyclonal antibodies, monoclonal antibodies, fragments thereof such as F(ab')2, and Fab fragments, as well as any naturally occurring or recombinantly produced binding partners, which are molecules that specifically bind a A$\beta$40, A$\beta$42 or tTau polypeptide. Any antibody fragment retaining the above criteria of a specific binding agent can be used. Antibodies are generated by state of the art procedures, e.g., as described in Tijssen (Tijssen, P., Practice and theory of enzyme immunoassays, Elsevier Science Publishers B.V., Amsterdam (1990), the whole book, especially pages 43-78). In addition, the skilled artisan is well aware of methods based on immunosorbents that can be used for the specific isolation of antibodies. By these means the quality of polyclonal antibodies and hence their performance in immunoassays can be enhanced (Tijssen, P., *supra*, pages 108-115).

For the achievements as disclosed in the present invention polyclonal antibodies raised in e.g. goats may be used. However, clearly also polyclonal antibodies from different species, e.g., rats, rabbits or guinea pigs, as well as monoclonal antibodies can be used. Since monoclonal antibodies can be produced in any amount required with constant properties, they represent ideal tools in development of an assay for clinical routine.

For measurement the sample obtained from an individual is incubated with the specific binding agent for the marker in question under conditions appropriate for formation of a binding agent marker-complex. Such conditions need not be specified, since the skilled artisan without any inventive effort can easily identify such appropriate incubation conditions. The amount of binding agent marker-complex is measured and used in the methods and uses of the invention. As the skilled artisan will appreciate there are numerous methods to measure the amount of the specific binding agent marker-complex all described in detail in relevant textbooks (cf., e.g., Tijssen P., *supra*, or Diamandis, E.P. and Christopoulos, T.K. (eds.), Immunoassay, Academic Press, Boston (1996)).

Particularly, monoclonal antibodies to the markers (Aβ40, Aβ42 and tTau) is used in quantitative (amount or concentration of the markers is determined) immunoassay.

Preferably, the marker in question is detected in a sandwich type assay format. In such assay a first specific binding agent is used to capture the marker in question on the one side and a second specific binding agent (e.g. a second antibody), which is labeled to be directly or indirectly detectable, is used on the other side. The second specific binding agent may contain a detectable reporter moiety or label such as an enzyme, dye, radionuclide, luminescent group, fluorescent group or biotin, or the like. Any reporter moiety or label could be used with the methods disclosed herein so long as the signal of such is directly related or proportional to the quantity of binding agent remaining on the support after wash. The amount of the second binding agent that remains bound to the solid support is then determined using a method appropriate for the specific detectable reporter moiety or label. For radioactive groups, scintillation counting or autoradiographic methods are generally appropriate. Antibody-enzyme conjugates can be prepared using a variety of coupling techniques (for review see, e.g., Scouten, W. H., Methods in Enzymology 135:30-65, 1987). Spectroscopic methods can be used to detect dyes (including, for example, colorimetric products of enzyme reactions), luminescent groups and fluorescent groups. Biotin can be detected using avidin or streptavidin, coupled to a different reporter group (commonly a radioactive or fluorescent group or an enzyme). Enzyme reporter groups can generally be detected by the addition of substrate (generally for a specific period of time), followed by spectroscopic, spectrophotometric or other analysis of the reaction products. Standards and standard additions can be used to determine the level of antigen in a sample, using well known techniques.

As described above, there is a variety of methods for measuring Aβ40 levels. An assay for Aβ40 specifically measures Aβ40, but not e.g., Aβ42 or Aβ43. The measurement of Aβ40 is typically based on binding specific for the COOH-terminus of the 1-40 Aβ sequence. Commercially available products for measuring Aβ40 include Amyloid beta 40 Human ELISA Kit (Thermo Fisher Scientific, Waltham, MA, USA), Simoa™ Aβ40 immunoassay (Quanterix Corporation, Lexington, MA, USA), and Amyloid β1-40 Assay Kit (Cisbo Assays, Codolet, France). In the Aβ40 assay used in the Examples, the specific monoclonal antibodies 23C2 binding to amino acids 25-40 of Aβ40 are used. Preferably, Aβ40 is measured using the Elesys® ECL technology on in an Elesys® measuring cell according to the manufacturer's instructions and as detailed in the Examples.

Also for the measurement of Aβ42 levels, there exist a variety of assays. Commercially available products for measuring Aβ42 include Amyloid beta 42 Human ELISA Kit (Thermo Fisher Scientific, Waltham, MA, USA), Simoa™ Aβ42 immunoassay (Quanterix Corporation, Lexington, MA, USA), INNOTEST® amyloid β(1-42) (Fujirebio, Gent, BE) and Elecsys® β-Amyloid (1-42) CSF assay (Id.No. 06986811-190; Roche Diagnostics AG; CH). In the Aβ42 assay from Roche Diagnostics, Germany, the specific monoclonal antibodies 21F12 and 3D6 are used. Preferably, Aβ42 is measured using the Elesys® ECL technology on in a Elesys® measuring cell according to the manufacturer's instructions and as detailed in the Examples.

The level of tTau may be determined with any suitable method. Commercially available products for measuring tTau include Tau (Total) Human ELISA Kit (Thermo Fisher Scientific, Waltham, MA, USA), Simoa™ Human Total Tau 2.0 immunoassay (Quanterix Corporation, Lexington, MA, USA), Total Tau ELISA Kit (human) (Dinova GmbH, Hamburg, DE) and Elecsys® Total-Tau CSF assay (Id.No. 07356994-190; Roche Diagnostics AG; CH). In the tTau assay from Roche Diagnostics, Germany, the specific monoclonal antibodies 5.28.464, 4.35.411 and are PC1C6 used. Preferably, tTau is measured using the Elesys® ECL technology on in an Elesys® measuring cell according to the manufacturer's instructions and as detailed in the Examples.

The step of measuring the level of a marker may be carried out as follows: The sample and optionally calibrator and/or control may be contacted with the binding agent (which could be immobilized, e.g. on a solid phase) under conditions allowing the binding of the agent to the marker. Optionally, unbound binding agents may be removed by a separation step (e.g. one or more washing steps). A second agent (e.g. a labeled agent) may be added to detect the bound binding agent to allow binding to and quantification of the same. Optionally, unbound second agent may be removed. The amount of the second binding agent which is proportional to the amount of the marker may be quantified, e.g. based on the label. Quantification may be done based on e.g. a calibration curve constructed for each assay by plotting measured value versus the concentration for each calibrator. The concentration or amount of marker in the sample may be then read from the calibration curve.

As a second step, the individual is identified as having or being at risk of developing an amyloid-positive dementia by comparing the combined value for the markers determined in step (a) to a control value, wherein an increased combined value relative to the control value is indicative of the amyloid-positive dementia. After the amount or concentration of the markers is measured (step a), the values of the amount or concentration of the marker molecules measured in step (a) are combined to obtain a combined value and the value obtained is compared to a control value. If the combined value for the markers determined in step (a) is increased relative to the control value, the individual is identified as having or being at risk of developing an amyloid-positive dementia.

"Combined to obtain a combined value" in the context of the present invention refers to a mathematical procedure. The combined value is calculated according to a mathematical operation, preferably an arithmetic operation using the amount/concentration of $A\beta40$, the amount/concentration of $A\beta42$ and the amount/concentration of tTau. The result of the operation is a value, namely the combined value. The combined value is compared to the combined value of the control, which has been obtained using the same mathematical procedure. In the invention usually either the amount or the concentration, preferably the concentration, of all markers is used in order to obtain the combined value for the individual and the control. Preferably, the combined value is obtained by adding the values obtained for the concentrations of the markers. In another preferred embodiment, the combined value is obtained by weighted calculation of the amount or concentration of the marker molecules in the samples. This means that the markers are given different weightings in the mathematical procedure.

For example, a logistic regression analysis may be performed with a binary outcome "amyloid-positive" and "amyloid-negative" as the dependent variable and the combination of $A\beta40$, $A\beta42$ and tTau as the independent variables. Classification accuracy may be assessed by Area under the ROC (Receiver-Operating Characteristic) curve (AUC) and sensitivity and specificity may be calculated (see below).

The control value may be obtained in different ways and using different test designs - as known to the skilled person. It is used to divide continuous results (here the combined values) into categories. In the present invention, the categories are positive (indicating an individual has or is at risk of developing an amyloid-positive dementia) and negative (indicating someone does not or is not at risk of developing an amyloid-positive dementia), wherein a value above the control results in the categorization "positive". Typically an external control is used (i.e. one or more samples not obtained from the individual tested).

In one embodiment of the invention, the control value is established using a control sample, which is obtained from an individual or preferably from a control population of individuals (also referred to as reference population) known to be free of a given condition, i.e. (an) amyloid-negative individual, which is preferably dementia-free. Alternatively, the control value is defined based on the analysis of a population of individuals known to have a certain condition (amyloid-positive dementia). Here the control value has to be chosen in order to be below the values typically determined for this population. Finally, the control value may be obtained by assessing a healthy population (amyloid-negative) and a diseased population (amyloid-positive dementia) and established a cut-off value in order to categorize an individual as having or being at risk of developing an amyloid-positive dementia or as amyloid-negative/healthy. It is within the skills of the practitioner to choose an appropriate control sample/population and/or a control value for the markers established therein. Exemplary populations are given in the Examples.

It will be appreciated by the skilled artisan that the absolute marker values established in a control will be dependent on the assay used. Preferably samples from 100 or more well-characterized individuals from the appropriate control/reference population are used to establish a control value. Also preferred the control/reference population may be chosen to consist of at least 20, 30, 50, 200, 500 or 1000 individuals. Healthy individuals represent a preferred reference population for establishing a control value.

In accordance with the present invention, an increased combined value is indicative of a future or present amyloid-positive dementia. If the value is increased the amyloid-positive dementia is already present or is likely to occur in future. The individual identified thereby may be subject to further diagnostic or therapeutic methods, including further blood tests, CSF tests, imaging methods like PET, behavioral studies or administration of a medicament. The skilled practitioner will be able to select suitable means in accordance with the medical practice of the prevailing country.

In one embodiment, the value is increased if the value amounts to at least 110%, more preferably by at least 120%, more preferably by at least 130%, more preferably by at least 140%, more preferably by at least 150%, more preferably by at least 160%, more preferably by at least 170%, more preferably by at least 180%, even more preferably by at least 190% relative to the control value.

In the present case three markers, namely $A\beta40$, $A\beta42$ and tTau, are used in the methods of the invention. Accordingly, a combined value is calculated using the amount/concentration of $A\beta40$, the amount/concentration of $A\beta42$ and the amount/concentration of tTau. The combined value is compared to the combined value of the control, which has been obtained using the same mathematical procedure.

In a preferred embodiment, the combined value is obtained by weighted calculation of the amount or concentration of the marker molecules in the sample. This means that each of the markers is given an individual weighting factor that may be higher or lower that that of the others.

In a more preferred embodiment, the combined value v is obtained by weighted calculation based of the level (amount or concentration) of Aβ40 ([Aβ40]), the level (amount or concentration) of Aβ42 ([Aβ42]) and the level (amount or concentration) of tTau ([tTau]) according to the equation:

$$v = a * [Aβ40] + b * [Aβ42] + c * [tTau] + d,$$

wherein a, b and c represent weighting factors and d represents an intercept. The intercept may be absent. Preferably, the weighting factors have been obtained by analyzing a reference population. A suitable procedure is described in the Examples.

In another preferred embodiment, the combined value v is obtained by weighted calculation based of the level (amount or concentration) of Aβ40 ([Aβ40]), the level (amount or concentration) of Aβ42 ([Aβ42]) and the level (amount or concentration) of tTau ([tTau]) according to the equation:

$$v = e * [Aβ42] / [Aβ40] + f * [tTau] + g,$$

wherein e and f represent the weighting factors and g represents an intercept. The intercept may be absent. Preferably, the weighting factors have been obtained by analyzing a reference population. A suitable procedure is described in the Examples.

For example, the combined value of Aβ40, Aβ42 and tTau is determined for each sample of a reference group and subsequent the median or a suitable cut-off of the combined values can be calculated as known to the skilled person. In (a) suitable reference population(s) one may calculate a regression model with a suitable outcome measurement (e.g. amyloid-positive dementia) as a dependent variable and using Aβ40, Aβ42 and tTau as independent variables.

The regression coefficients delivered by the regression model for Aβ40, Aβ42 and tTau can then be used as weighting factors i) a, b and c or ii) e and f and as intercept i) d or ii) g, respectively in order to calculate the combined value v for each individual in the population(s). On these combined v values the control, e.g. a cut-off can be calculated, such as the median of v in the population(s).

Individuals with v values above the control value or cut-off would be considered as having or being at risk of developing an amyloid-positive dementia and individuals at or below the control or cut-off as not having or being at risk of developing an amyloid-positive dementia.

The above statistical methods are only examples of statistic methods for the identification of amyloid-positive individuals. The skilled statistician will know suitable methods for analyzing the prevailing data and then providing a suitable control value or cut-off.

Accordingly, in a preferred embodiment, the individual is identified as having or being at risk of developing an amyloid-positive dementia if the combined value for the markers is above a cut-off value. The values for the markers Aβ40, Aβ42 and tTau as measured in a reference population or reference populations are used to establish a cut-off value. A value above such cut-off value is considered as being indicative for an individual having or being at risk of developing an amyloid-positive dementia. In one embodiment a fixed cut-off value is or was established prior to the testing of the individual. Such cut-off value is chosen to match the diagnostic question of interest. Preferable, the cut-off is the median of the reference populations comprising amyloid-positive and amyloid-negative Individuals. A suitable median or cut-off value may be chosen depending on how many individuals one would wish to identify as having or being at risk of developing an amyloid-positive dementia. A higher number of individuals selected for "amyloid-positive dementia" decreases the risk to falsely characterize a diseased or risk individual as healthy and therefore increases sensitivity (true positive rate). On the other hand it increases the risk to falsely characterize an individual as having or being at risk of developing an amyloid-positive dementia and therefore decreases specificity (true negative rate). For a lower number of selected individuals the same is true vice versa. For any test, there is usually a trade-off between the measures. For instance, in an airport security setting in which one is testing for potential threats to safety, scanners may be set to trigger on low-risk items like belt buckles and keys (low specificity), in order to reduce the risk of missing objects that do pose a threat to the aircraft and those aboard (high sensitivity). This trade-off can be represented graphically as a receiver operating characteristic curve (see below). A perfect predictor would be described as 100% sensitive (e.g., all sick are identified as sick) and 100% specific (e.g., all healthy are not identified as sick); however, theoretically any predictor will possess a minimum error bound known as the Bayes error rate. The cut-off can be set in order to either increase sensitivity or specificity.

In statistics, a receiver operating characteristic (ROC), or ROC curve, is a graphical plot that illustrates the performance of a binary classifier system as its discrimination threshold is varied. The curve is created by plotting the true positive rate (TPR) against the false positive rate (FPR) at various threshold settings. As detailed above, the true-positive rate is also known as sensitivity or the sensitivity index d', known as "d-prime" in signal detection and biomedical informatics, or recall in machine learning. The false-positive rate is also known as the fall-out and can be calculated as (1 - specificity). The ROC curve compares sensitivity versus specificity across a range of values for the ability to predictor a dichotomous outcome. The area under the curve (AUC presents the overall accuracy for comparing test performance (Florkowski CM, 2008, Clin Biochem Rev 29(Suppl 1): S83-S87). Sensitivity is the ability of a test to correctly classify an individual as diseased. The ability of a test to correctly classify an individual as disease-free is called specificity (Fawcett T, 2006, Pattern Recognition Letters 27: 861-874).

ROC analysis provides tools to select possibly optimal models and to discard suboptimal ones independently from (and prior to specifying) the cost context or the class distribution. ROC analysis is related in a direct and natural way to cost/benefit analysis of diagnostic decision making.

As detailed above, the weighting factors for may be obtained by analyzing one or more reference population(s). Accordingly, in a preferred embodiment, the weighting factors have been obtained by analyzing one or more reference population(s). In accordance with the above, the control value may have been obtained from an amyloid-negative control population or an amyloid-positive control population or a combination thereof, i.e. an amyloid-negative control population and an amyloid-positive control population.

As detailed above, AD-related dementia is associated with amyloid beta deposits in the brain. The percentage of individuals showing amyloid beta deposits and the brain area affected increases with progression of the disease. Accordingly, there is a very small portion of amyloid-positive individuals which do not yet show any AD symptoms, which are cognitively normal (CN) and which are regarded as at-risk individuals. The percentage increases for individuals with subjective cognitive decline (SCD) and mild cognitive impairment (MCI). Finally, AD is per definition characterized by amyloid plaques and therefore an amyloid-positive dementia. It can be part of mixed dementia, e.g. in combination with vascular dementia, Lewy body dementia and/or fronto-temporal lobe dementia. Accordingly, in a preferred embodiment, the individual is cognitively normal (CN), has a subjective cognitive decline (SCD), has a mild cognitive impairment (MCI) or has Alzheimer's disease (AD), optionally AD combined with a other type of dementia (mixed dementia), particularly mixed with vascular dementia, Lewy body dementia and/or fronto-temporal lobe dementia.

As detailed above, a sample analyzed in the present invention may be any blood sample suitable for measuring the markers according to the present invention and is not a brain sample or a cerebrospinal fluid sample. For use in the present invention, blood may be drawn from a vein, usually from the inside of the elbow or the back of the hand. The blood may be collected e.g. into a pipette, or onto a slide or test strip. Accordingly, in the present invention, the sample obtained from the individual is a blood sample, particularly selected from the group consisting of serum, plasma, and whole blood, especially plasma.

As detailed above, the marker molecules may be measured by different methods and at different levels. Preferably, the marker molecule(s) is/are measured at the protein level.

As detailed above, the individual according to the present invention may be any human or non-human animal, particularly a mammal, especially a human. Thus, the methods and uses described herein are applicable to both human and veterinary disease. Evidently, non-human mammals of particular interest include domestic animals, pets, and animals of commercial value (e.g. domestic animals such as horses) or personal value (e.g. pets such as dogs, cats). The method is especially preferred with human individuals, for which diagnostic methods are commonly employed. In a particularly preferred embodiment the individual is therefore a human.

After the individual has been identified as having or being at risk of developing an amyloid-positive dementia, suitable steps may be recommended or taken. The diagnosis could be confirmed by a liquor test or a brain positron-emission tomography (PET) analysis. Furthermore, steps for preventing the outbreak of the dementia, for preventing worsening of the dementia or for ameliorating the symptoms of the disease. Also regular controls or monitoring of the individual's state or further diagnostic tests may be suitable. Preventive means may include administration of non-steroidal anti-inflammatory drugs, avoidance of hormone replacement therapy in menopause, change in lifestyle (e.g. more intellectual activities or suitable diet). Medications for cognitive problems associated with AD include acetylcholinesterase inhibitors (such as terrine, rivastigmine, galantamine and donepezil) and NMDA receptor antagonists (memantine) is an. Atypical antipsychotics are modestly useful in reducing aggression and psychosis in people with Alzheimer's disease. Huperzine A may be also helpful. Psychosocial inventions may be used alone or in combination with other treatments.

In a second aspect, the present invention relates to the use of Aβ40, Aβ42 and tTau as marker combination for the identification of an individual having or being at risk of developing an amyloid-positive dementia, wherein the detection of an increased combined value of the marker combination in a sample obtained from the individual as compared to the combined value as established in one or more reference population(s) (control value) is indicative of the amyloid-positive dementia, wherein the sample is not a brain sample or a cerebrospinal fluid sample, but a blood sample.

The use according to the second aspect may be further defined as specified for the method of the first aspect of the present invention. Particularly, with respect to the terms used in the second aspect of the present disclosure it is referred to the terms, examples and specific embodiments used in the first of the present disclosure, which are also applicable to the second aspect of the present disclosure.

Also disclosed herein is a method for detecting an individual with an increased value for a marker combination, the method comprising

a) measuring in a sample obtained from the individual the amount or concentration of the marker molecules A$\beta$40, A$\beta$42 and tTau, and

b) detecting an increased combined value for the markers determined in step (a) relative to the combined value as established in one or more reference population(s) (control value),

wherein the sample is not a brain sample or a cerebrospinal fluid sample.

The method may be further defined as specified for the method of the first aspect of the present invention. Particularly, with respect to the terms used in the method, it is referred to the terms, examples and specific embodiments used in the first aspect of the present disclosure, which are also applicable.

In general, the disclosure is not limited to the particular methodology, protocols, and reagents described herein because they may vary. Further, the terminology used herein is for the purpose of describing particular embodiments only and is not intended to limit the scope of the present disclosure. As used herein and in the appended claims, the singular forms "a", "an", and "the" include plural reference unless the context clearly dictates otherwise. Similarly, the words "comprise", "contain" and "encompass" are to be interpreted inclusively rather than exclusively.

Unless defined otherwise, all technical and scientific terms and any acronyms used herein have the same meanings as commonly understood by one of ordinary skill in the art in the field of the disclosure. Although any methods and materials similar or equivalent to those described herein can be used in the practice as presented herein, the specific methods, and materials are described herein.

The disclosure is further illustrated by the following figures and examples, although it will be understood that the figures and examples are included merely for purposes of illustration and are not intended to limit the scope of the disclosure unless otherwise specifically indicated.

## FIGURES

**Figures 1 to 4** illustrate ROC curves for 4 different populations of human individuals

Figure 1: Population 1: Cognitive normal, neurological diseased and patients with different types of dementia as defined in Example 1

Figure 2: Population 2: Cognitive normals and patients with different stages of Alzheimer's disease as defined in Example 2

Figure 3: Population 3: Cognitive normals and patients with mild cognitive impairment as defined in Example 3

Figure 4: Population 4: Cognitive normals and patients with mild cognitive impairment or mild Alzheimer's disease as defined in Example 4

## EXAMPLES

### Example 1: General description of the Elecsys® sandwich assays

The Elecsys® sandwich assay is an immune-assay performed on the Elecsys® system. It is based on the formation of the antibody-antigen-antibody sandwich and its binding to streptavidin-coated microparticles. A first and a second monoclonal antibody are used to bind to the target antigen of interest ($\beta$40, A$\beta$42 or tTau) (incubation time used: 9 min). The first antibody is biotinylated and the second antibody is ruthenium-labelled ruthenylated. Additionally, a streptavidin-coated microparticle is present (using addition in a second incubation step lasting for 9 min). Upon binding of the two antibodies to the target antigen and the binding of streptavidin to biotin, a complex comprising: microparticle - 1st antibody - target - 2nd antibody - ruthenium is formed, which may be immobilized in the detection device (e.g. by applying a magnetic field, when the microparticle is paramagnetic), particularly on the surface of an electrode using the microparticle, and which may be detected in a measuring cell based on chemiluminescence using the ruthenium label.

### 1.1 CSF assays

### 1.1.1 Elecsys® Total-Tau CSF assay

The Elecsys® Total-Tau CSF assay (Id.No. 07356994-190; Roche Diagnostics AG; CH) was used for the determination of tTau in cerebrospinal fluid (CSF) samples.

**1st incubation** (9 minutes): 50 $\mu$L of sample, two biotinylated monoclonal Tau-specific antibodies (5.28.464 and 4.35.411; Roche Diagnostics AG; CH) and a monoclonal Tau-specific antibody (PC1C6; MAB3420; e.g. available from Merck KGaA, DE) labeled with a ruthenium complex (Tris(2,2'-bipyridyl)ruthenium(II)-complex; Ru(bpy)) were co-incubated and formed a sandwich complex comprising the two biotinylated antibodies, tTau and the ruthenylated antibody.

**2nd incubation** (9 min): Streptavidin-coated microparticles (Elecsys® beads) were added to the mixture of the first incubation step and during this second incubation the complex comprising the biotinylated antibodies, tau and the ruthenylated antibody became bound to the solid phase via interaction of biotin and streptavidin.

**Measurement:** The reaction mixture was aspirated into the measuring cell where the microparticles were magnetically captured onto the surface of the electrode. Unbound substances were then removed with ProCell/ProCell M. Application of a voltage to the electrode then induces chemiluminescent emission which was measured by a photomultiplier. Results were determined via a calibration curve which is an instrument specifically generated by 2-point calibration and a master curve provided via the reagent barcode or e-barcode.

### 1.1.2 Elecsys® β-Amyloid (1-42) CSF assay

The Elecsys® β-Amyloid (1-42) CSF assay (Id.No. 06986811-190; Roche Diagnostics AG; CH) was used for the determination of Aβ42 in cerebrospinal fluid samples.

**1st incubation** (9 min): 50 μL of a sample, a biotinylated monoclonal β-Amyloid (1-42) specific antibody (21F12) and a monoclonal β-Amyloid-specific antibody (3D6) labeled with a ruthenium complex were co-incubated and form a sandwich complex comprising the biotinylated antibody, Aβ42 and the ruthenylated antibody.

**2nd incubation** (9 min): Streptavidin-coated microparticles (Elecsys® beads) were added to the mixture of the first incubation step and during this second incubation the complex comprising the biotinylated antibody, Aβ42 and the ruthenylated antibody became bound to the solid phase via interaction of biotin and streptavidin

**Measurement:** The reaction mixture was aspirated into the measuring cell where the microparticles are magnetically captured onto the surface of the electrode. Unbound substances were then removed with ProCell/ProCell M. Application of a voltage to the electrode then induces chemiluminescent emission which was measured by a photomultiplier. Results were determined via a calibration curve which is an instrument specifically generated by 2-point calibration and a master curve provided via the reagent barcode or e-barcode.

### 1.2. Plasma Assays

### 1.2.1 Elecsys® Total-Tau Plasma assay

For this plasma application the same reagents as for the CSF assay (see 1.1.1) were used:

- R1 = liquid, ready to use reagent, which contains the biotinylated antibodies
- R2 = liquid, ready to use reagent, which contains the Ru-labeled antibody
- M = liquid, ready to use reagent, which contains the Streptavidin coated micro particles

In comparison to the CSF assay only other calibrators and controls were used to overcome the different sample matrix and the different analyte levels in plasma. A chemically synthesized tau-antigen (same as for the CSF-assay) was spiked into a protein-containing TRIS-buffer in concentrations of approx. 0, 30, 100, 500 and 5000 pg/ml. These calibrators were frozen at -80°C before use.

**1st incubation, 2nd incubation and Measurement** were carried out as detailed under 1.1.1 with the exception that the results were determined via a calibration curve which is generated by a 5-point calibration curve.

### 1.2.2 Elecsys® β-Amyloid (1-42) Plasma assay

For this plasma application the same reagents as for the CSF assay (see 1.1.2) were used:

- R1 = liquid, ready to use reagent, which contains the biotinylated antibody
- R2 = liquid, ready to use reagent, which contains the Ru-labeled antibody
- M = liquid, ready to use reagent, which contains the Streptavidin coated micro particles

In comparison to the CSF assay only other calibrators and controls were used to overcome the different sample matrix and the different analyte levels in plasma. Chemically synthesized Aβ42-antigen (same as for the CSF asaay) was spiked into horse serum in concentrations of approx. 0, 20, 50, 250 and 1200 pg/ml. These calibrators were frozen at -80°C before use.

**1st incubation, 2nd incubation and Measurement** were carried out as detailed under 1.1.2 with the exception that the results were determined via a calibration curve which is generated by a 5-point calibration curve.

### 1.2.3 Elecsys® β-Amyloid (1-40) Plasma assay

**1st incubation** (9 minutes): 50 $\mu$L of sample, a biotinylated monoclonal Aß40-specific antibodies (23C2) and a monoclonal β-Amyloid-specific antibody (3D6) labeled with a ruthenium complex were co-incubated and form a sandwich complex comprising the biotinylated antibody, Aß40 and the ruthenylated antibody.

**2nd incubation** (9 min): Streptavidin-coated microparticles (Elecsys® beads) were added to the mixture of the first incubation step and during this second incubation the complex comprising the biotinylated antibody, Aß40 and the ruthenylated antibody became bound to the solid phase via interaction of biotin and streptavidin.

**Measurement:** The reaction mixture was aspirated into the measuring cell where the microparticles were magnetically captured onto the surface of the electrode. Unbound substances were then removed with ProCell/ProCell M. Application of a voltage to the electrode then induced chemiluminescent emission which was measured by a photomultiplier. Results were determined via a calibration curve which is generated by a 5-point calibration curve.

### Calibrators and Controls

Phosphate-buffered saline containing 0.4 g/L BSA was spiked with a chemically synthesized Aß-40 antigen, which contained both antibody recognizing epitopes 1-12 and 25-40. Following levels of Aß40 were produced: 0, 250, 500, 2500 and 10.000 pg/mL.

### Example 2: Sample collective

A large sample collective consisting of patients with Alzheimer Disease (AD) other dementias like frontotemporal dementia etc., Mild Cognitive Impairment (MCI), neurological diseases and also cognitive normals were prospectively collected some years ago. From all patients liquor and also EDTA-plasma was collected in parallel at the same time point. Since no PET imaging was available for the determination of brain amyloid-ß-positive or -negative status, we measured the CSF samples with the immunoassays ß-Amyloid(1-42) and tTAU from Roche Diagnostics.

We used the tTAU/Abeta42 ratio in CSF with a cut-off value of 0.28 as reference to classify the samples into amyloid-positive (>0.28) and amyloid-negative (<0.28). This ratio showed a high concordance with the amyloid PET visual read out with an AUC of 94% (see the method sheet of tTAU assay Id. No. 07356994 190; Roche Diagnostics AG, CH).

Following patient samples were measured:

| Clinical diagnosis | Amyloid- | Amyloid+ |
|---|---|---|
| AD (MMSE > 22) | 20 (19.6%) | 82 (80.4%) |
| AD (MMSE 14-22) | 9 (6.9%) | 121 (93.1%) |
| CN (cognitive normal) | 31 (86.1%) | 5 (13.9%) |
| MCI (mild cognitive impairment) | 66 (58.4%) | 47 (41.6%) |
| FTD (fronto-temporal lobe dementia) | 13 (39.4%) | 20 (60.6%) |
| Lewy body dementia | 0 (0%) | 1 (100%) |
| Mixed dementia | 12 (23.1%) | 40 (76.9%) |
| Vascular dementia | 15 (31.9%) | 32 (68.1%) |
| Neurological disease | 84 (77.1%) | 25 (22.9%) |
| Total | 250 | 373 |

For the definition of amyloid positive/negative we used the tTau/Abeta42 ratio in CSF with cut-off value of 0.28 as reference to classify samples into amyloid+ (>0.28) and amyloid- (<0.28).

### Example 3: Data analysis

The predictive power was compared by using different models. The most basic "model" is the ratio of two or more biomarkers (dividing the value of one biomarker by the value of the other). To allow for non-linear relationships between two or more markers, we introduced logistic regression models with different marker combinations. More specifically, we compared the following ratios and models:

- Ab42/Ab40 ratio (reference)
- tTau/Ab42 ratio
- Ab42/Ab40 (ratio) + tTau (model 2; weighted calculation)
- Ab42 + Ab40 + tTau (model 1; weighted calculation)

Logistic regression (or logit regression) is a statistical method to estimate a model that describes a binary dependent variable. The log-odds of the probability of an event is a linear combination of the predictor variables. The estimated response is the probability of a binary response (here amyloid+/-) based on predictor variables (here: the biomarkers). It can be used as a classifier to predict classes of new samples based on their independent variable levels.

The model can be interpreted the following way:

$$logit(p) = \beta_0 + \beta_1 X_1 + \beta_2 X_2 + \beta_3 X_3$$

with $p$ being the probability of being amyloid+, X being the predictive biomarkers (plasma Ab42, plasma Ab40, plasma tTau, or plasma ratios) and $\beta$ being the coefficients (also referred to as weighting factor).

Then,

$$odds = \frac{p}{1-p} = \frac{probability(amyloid+)}{probability(amyloid-)}$$

and

$$logit(p) = ln(\frac{p}{1-p})$$

For the analysis involving all three biomarkers Ab42, Ab40 and tTau, weighting factors (a to e) were calculated using the following equations:

$$v = a * [A\beta40] + b * [A\beta42] + c * [tTau] + d \text{ (model 1)}$$

$$v = e * [A\beta42] / [A\beta40] + f * [tTau] + g \text{ (model 2)}$$

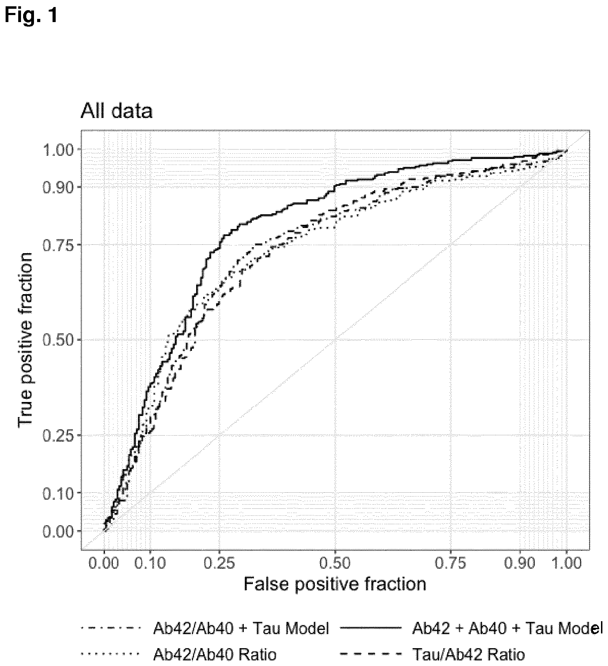

Additionally, receiver operating characteristic (ROC) curves have been established by plotting the true positive rate against the false positive rate (see Figures 1 to 4). The true-positive rate is also known as sensitivity. The false-positive rate is also known as the fall-out or probability of false alarm and can be calculated as (1 - specificity). The area under the curve (AUC) was determined to characterize the test.

## Example 4: Results

### 1. Population 1: All measured patients: Cognitive normals, neurological diseased, and patients with different types of dementia

A population of 623 humans (373 amyloid-positives and 250 amyloid negatives), which was composed as indicated in following Table 1a, was used in a first data analysis:

| Table 1a | CSF: amyloid - | CSF: amyloid+ |
|---|---|---|
| Diagnosis | | |
| Total (non-NA) | 250 | 373 |
| AD (MMSE>22) | 20 (19.6%) | 82 (80.4%) |
| AD (MMSE22-14) | 9 (6.9%) | 121 (93.1%) |
| cognitive normal | 31 (86.1%) | 5 (13.9%) |
| Fronto-temporal lobe dementia | 13 (39.4%) | 20 (60.6%) |
| Lewy body dementia | 0 (0%) | 1 (100%) |

(continued)

| Table 1a | CSF: amyloid - | CSF: amyloid+ |
|---|---|---|
| Mild cognitive impairment | 66 (58.4%) | 47 (41.6%) |
| Mixed dementia | 12 (23.1%) | 40 (76.9%) |
| neurological disease | 84 (77.1%) | 25 (22.9%) |
| Vascular dementia | 15 (31.9%) | 32 (68.1%) |

The population includes cognitive normals (non-NA) as well as patients with different types of dementia, as indicated above. The results of the analysis are summarized in the following Table 1 b:

| Table 1b | Model 1: Ab42 + Ab40 + tTau | Model 2: Ab42/Ab40 + tTau |
|---|---|---|
| (Intercept) | 0.97* (0.46) | 1.80** (0.68) |
| c (TAU_Elecsys_PLASMA) | 0.11*** (0.02) | 0.09*** (0.02) |
| b (AB42_Elecsys_PLASMA) | -0.25*** (0.03) | |
| a (AB40_Elecsys_PLASMA) | 11.04*** (1.91) | |
| d (AB42_AB40_Elecsys_PLASMA) | | -43.19***(8.80) |
| ***$p < 0.001$, **$p < 0.01$, *$p < 0.05$ | | |

Accordingly, model 1 (Ab42 + Ab40 + tTau) can be written as

$$\text{logit}(p) = 0.97 + 0.11 * [\text{tTau}] - 0.25 * [\text{A}\beta42] + 11.04 * [\text{A}\beta40]$$

and model 2 (Ab42 / Ab40 + tTau) can be written as

$$\text{logit}(p) = 1.80 + 0.09 * [\text{tTau}] - 43,19 * [\text{A}\beta42] / [\text{A}\beta40]$$

The quality of the analysis for the four different marker combinations can be characterized by the sensitivity and specificity as shown in Figure 1 and specified in Table 1 c:

| Table 1c | AUC | Specificity | |
|---|---|---|---|
| | | Sensitivity = 0.8 | Sensitivity = 0.85 |
| Ab42/Ab40 | 0.73 | 0.50 | 0.41 |
| tTau/Ab42 | 0.73 | 0.56 | 0.48 |
| Ab42/Ab40 + tTau | 0.73 | 0.55 | 0.43 |
| Ab42 + Ab40 + tTau | 0.79 | 0.71 | 0.60 |

Evidently, the presently used standard combination of markers (Ab42/Ab40) can be improved. The marker combinations of Aβ42, Aβ40 and tTau are superior over the standard combination, with model 1 giving the best results.

**2. Population 2: Cognitive normals and patients with different stages of Alzheimer's disease**

A population of 381 humans, which was composed as indicated in following Table 2a, was used in a second data analysis:

| Table 2a | CSF: amyloid - | CSF: amyloid+ |
|---|---|---|
| Diagnosis | | |
| Total | 126 | 255 |
| AD (MMSE>22) | 20 (19.6%) | 82 (80.4%) |
| AD (MMSE22-14) | 9 (6.9%) | 121 (93.1%) |
| cognitive normal | 31 (86.1%) | 5 (13.9%) |

(continued)

| Table 2a | CSF: amyloid - | CSF: amyloid+ |
|---|---|---|
| Mild cognitive impairment | 66 (58.4%) | 47 (41.6%) |

The population includes cognitive normals as well as with different stages of Alzheimer's disease, but excludes other forms of dementia and neurological diseases. The results of the analysis are summarized in the following Table 2b:

| Table 2b | Model 1: Ab42 + Ab40 + tTau | Model 2: Ab42/Ab40 + tTau |
|---|---|---|
| (Intercept) | 2.33*** (0.69) | 3.70*** (0.99) |
| TAU_Elecsys_PLASMA | 0.13*** (0.03) | 0.09** (0.03) |
| AB42_Elecsys_PLASMA | -0.39*** (0.05) | |
| AB40_Elecsys_PLASMA | 16.95*** (2.96) | |
| AB42_AB40_Elecsys_PLASMA | | 66.44*** (13.44) |
| ***$p < 0.001$, **$p < 0.01$, *$p < 0.05$ | | |

Accordingly, model 1 (Ab42 + Ab40 + tTau) can be written as

$$logit(p) = 2.33 + 0.13 * [tTau] - 0.39 * [A\beta42] + 16.95 * [A\beta40]$$

and model 2 (Ab42 / Ab40 + tTau) can be written as

$$logit(p) = 3.70 + 0.09 * [tTau] + 66.44 * [A\beta42] / [A\beta40]$$

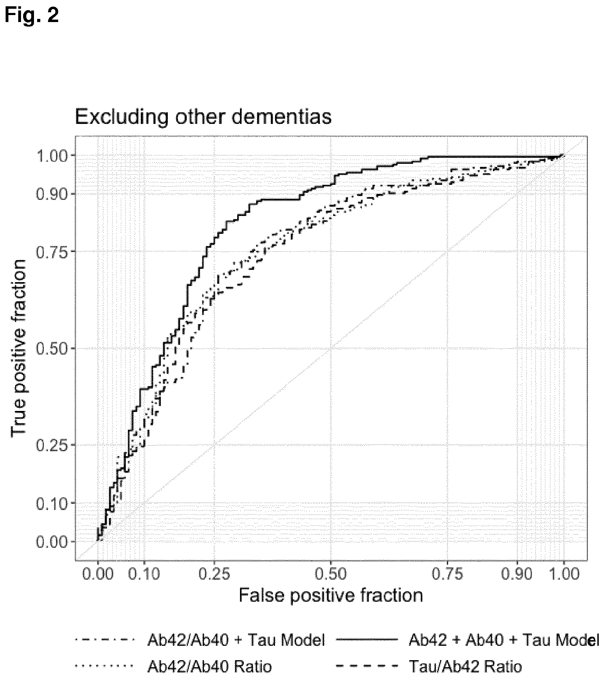

The quality of the analysis for the four different marker combinations can be characterized by the sensitivity and specificity as shown in Figure 2 and specified in Table 2c:

| Table 2c | AUC | Specificity | |
|---|---|---|---|
| | | Sensitivity = 0.8 | Sensitivity = 0.85 |
| Ab42/Ab40 | 0.75 | 0.58 | 0.49 |
| tTau/Ab42 | 0.74 | 0.57 | 0.51 |
| Ab42/Ab40 + tTau | 0.75 | 0.61 | 0.53 |
| Ab42 + Ab40 + tTau | 0.82 | 0.73 | 0.68 |

Evidently, the presently used standard combination of markers (Ab42/Ab40) can be improved. The marker combinations of A$\beta$42, A$\beta$40 and tTau are superior over the standard combination, with model 1 giving the best results. It is assumed that the analysis of a population excluding patients with other dementias may lead to better results as in population 1 dementia patients may have been allocated to false dementia groups in the dementia diagnosis. This assumption is based on the fact that the cerebrospinal fluid samples of patients suffering from amyloid-negative dementias has been found amyloid-positive (see Table 1a).

### 3. Population 3: Cognitive normals and patients with mild cognitive

A population of 151 humans, which was composed as indicated in following Table 3a, was used in a third data analysis:

| Table 3a | CSF: amyloid - | CSF: amyloid+ |
|---|---|---|
| Diagnosis | | |
| Total | 97 | 52 |
| cognitive normal | 31 (86.1%) | 5 (13.9%) |
| Mild cognitive impairment | 66 (58.4%) | 47 (41.6%) |

The population includes cognitive normals as well as patients with mild cognitive impairment, as indicated above. The results of the analysis are summarized in the following Table 3b:

| Table 3b | Model 1: Ab42 + Ab40 + tTau | Model 2: Ab42/Ab40 + tTau |
|---|---|---|
| (Intercept) | 2.69** (1.03) | 4.50** (1.67) |
| TAU_Elecsys_PLASMA | 0.03 (0.04) | -0.00 (0.04) |
| AB42_Elecsys_PLASMA | -0.38*** (0.08) | |
| AB40_Elecsys_PLASMA | 16.28*** (4.17) | |
| AB42_AB40_Elecsys_PLASMA | | 76.09*** (22.13) |
| ***$p < 0.001$, **$p < 0.01$, *$p < 0.05$ | | |

Accordingly, model 1 (Ab42 + Ab40 + tTau) can be written as

$$\text{logit}(p) = 2.69 + 0.03 * [tTau] - 0.38 * [A\beta42] + 16.28 * [A\beta40]$$

and model 2 (Ab42 / Ab40 + tTau) can be written as

$$\text{logit}(p) = 4.50 + 0 * [tTau] + 76.09 * [A\beta42] / [A\beta40]$$

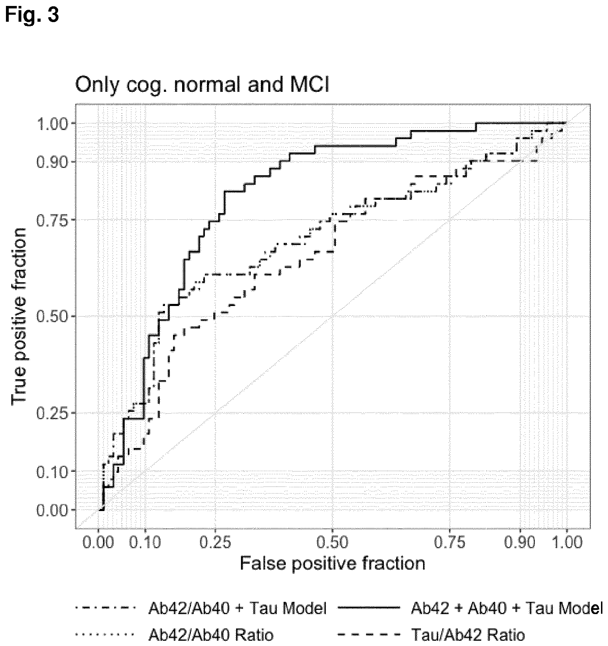

The quality of the analysis for the four different marker combinations can be characterized by the sensitivity and specificity as shown in Figure 3 and specified in Table 3c:

| Table 3c | AUC | Specificity | |
|---|---|---|---|
| | | Sensitivity = 0.8 | Sensitivity = 0.85 |
| Ab42/Ab40 | 0.70 | 0.41 | 0.25 |
| tTau/Ab42 | 0.65 | 0.43 | 0.32 |
| Ab42/Ab40 + tTau | 0.70 | 0.41 | 0.26 |
| Ab42 + Ab40 + tTau | 0.81 | 0.73 | 0.67 |

Evidently, the presently used standard combination of markers (Ab42/Ab40) can be improved. The marker combinations of Aβ42, Aβ40 and tTau are superior over the standard combination, with model 1 giving the best results. Population 3 is of particular diagnostic interest in the detection of MCI as very early stage of Alzheimer's disease.

**4. Population 4: Cognitive normals and patients with mild cognitive or mild Alzheimer's disease**

A population of 251 humans, which was composed as indicated in following Table 4a, was used in a fourth data analysis:

| Table 4a | CSF: amyloid - | CSF: amyloid+ |
|---|---|---|
| Diagnosis | | |
| Total | 117 | 134 |
| AD (MMSE>22) | 20 (19.6%) | 82 (80.4%) |
| Cognitive normal | 31 (86.1%) | 5 (13.9%) |
| Mild cognitive impairment | 66 (58.4%) | 47 (41.6%) |

The population includes cognitive normals (non-NA) as well as patients with mild cognitive impairment or mild Alzheimer's disease, as indicated above. The results of the analysis are summarized in the following Table 4b:

| Table 4b | Model 1: Ab42 + Ab40 + tTau | Model 2: Ab42/Ab40 + tTau |
|---|---|---|
| (Intercept) | 3.00*** (0.84) | 5.13*** (1.24) |

(continued)

| Table 4b | Model 1: Ab42 + Ab40 + tTau | Model 2: Ab42/Ab40 + tTau |
|---|---|---|
| TAU_Elecsys_PLASMA | 0.06 (0.04) | 0.04 (0.03) |
| AB42_Elecsys_PLASMA | -0.46*** (0.07) | |
| AB40_Elecsys_PLASMA | 20.90*** (3.62) | |
| AB42_AB40_Elecsys_PLASMA | | 85.46*** (16.89) |
| ***$p < 0.001$, **$p < 0.01$, *$p < 0.05$ | | |

Accordingly, model 1 (Ab42 + Ab40 + tTau) can be written as

$$\text{logit}(p) = 3.00 + 0.06 * [\text{tTau}] - 0.46 * [A\beta 42] + 20.90 * [A\beta 40]$$

and model 2 (Ab42 / Ab40 + tTau) can be written as

$$\text{logit}(p) = 5.13 + 0.04 * [\text{tTau}] + 85.46 * [A\beta 42] / [A\beta 40]$$

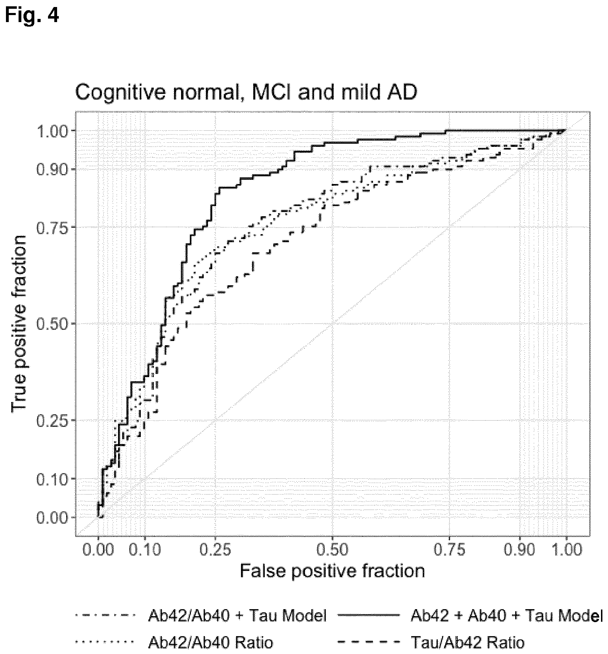

The quality of the analysis for the four different marker combinations can be characterized by the sensitivity and specificity as shown in Figure 4 and specified in Table 4c:

| Table 4c | AUC | Specificity | |
|---|---|---|---|
| | | Sensitivity = 0.8 | Sensitivity = 0.85 |
| Ab42/Ab40 | 0.76 | 0.55 | 0.46 |
| tTau/Ab42 | 0.71 | 0.52 | 0.41 |
| Ab42/Ab40 + tTau | 0.76 | 0.58 | 0.50 |
| Ab42 + Ab40 + tTau | 0.84 | 0.76 | 0.74 |

Evidently, the presently used standard combination of markers (Ab42/Ab40) can be improved. The marker combinations of Aβ42, Aβ40 and tTau are superior over the standard combination, with model 1 giving the best results. Population 4 is of particular diagnostic interest in the detection of early stages of AD including MCI.

## 5. Summary Table

| Table 5 | AUC | Sensitivity | Specificity |
|---|---|---|---|
| Population 1: AD versus controls (all) | | | |
| Aß42/Aß40 | 0.73 | 80 % | 50 % |
| Tau/Aß42 | 0.73 | 80 % | 56 % |
| tTau + Aß42/Aß40 | 0.73 | 80 % | 55 % |
| tTau + Aß42 + Aß 40 | 0.79 | 80 % | 71 % |
| Population 2: AD versus control (no dementia) | | | |
| Aß42/Aß40 | 0.75 | 80 % | 58 % |
| Tau/Aß42 | 0.74 | 80 % | 57 % |
| tTau + Aß42/Aß40 | 0.75 | 80 % | 61 % |
| Tau + Aß42 + Aß 40 model | 0.82 | 80 % | 73 % |
| Population 3: MCI versus control | | | |
| Aß42/Aß40 | 0.70 | 80 % | 41 % |
| Tau/Aß42 | 0.65 | 80 % | 43 % |
| tTau + Aß42/Aß40 | 0.70 | 80 % | 41 % |
| Tau + Aß42 + Aß 40 model | 0.81 | 80 % | 73 % |

(continued)

| Population 4: MCI / mild AD versus control | | | |
|---|---|---|---|
| Aß42/Aß40 | 0.76 | 80 % | 55 % |
| Tau/Aß42 | 0.71 | 80 % | 52 % |
| tTau + Aß42/Aß40 | 0.76 | 80 % | 58 % |
| Tau + Aß42 + Aß 40 model | 0.84 | 80 % | 76 % |

## Claims

1. A method of identifying an individual as having or being at risk of developing an amyloid-positive dementia, the method comprising

   a) measuring in a sample obtained from the individual the amount or concentration of the marker molecules amyloid $\beta40$ (A$\beta40$), amyloid $\beta42$ (A$\beta42$) and total Tau (tTau), and
   b) identifying an individual as having or being at risk of developing an amyloid-positive dementia by comparing the combined value for the markers determined in step (a) to a control value, wherein an increased combined value relative to the control value is indicative of the amyloid-positive dementia,

   wherein the sample is a blood sample.

2. The method of claim 1, wherein the combined value is obtained by weighted calculation of the amount or concentration of the marker molecules in the samples.

3. The method of claim 2, wherein the combined value v is obtained by weighted calculation based of the level (amount or concentration) of A$\beta40$ ([A$\beta40$]), the level (amount or concentration) of A$\beta42$ ([A$\beta42$]) and the level (amount or concentration) of tTau ([tTau]) according to the equation:

$$v = a * [A\beta40] + b * [A\beta42] + c * [tTau] + d$$

   wherein a, b and c represent the weighting factors, and d represents the intercept.

4. The method of any of claims 1 to 3, wherein the individual is identified as having or being at risk of developing an amyloid-positive dementia if the combined value for the markers is above a cut-off value.

5. The method of claim 2 or 3, wherein the weighting factors have been obtained by analyzing one or more reference population(s).

6. The method of any of claims 1 to 5, wherein, the control value has been obtained from an amyloid-negative control population or an amyloid-positive control population or a combination thereof.

7. The method of any of claims 1 to 6, wherein the individual is cognitively normal (CN), has a subjective cognitive decline (SCD), has a mild cognitive impairment (MCI) or has Alzheimer's disease (AD), optionally AD combined with a other type of dementia (mixed dementia), particularly mixed with vascular dementia, Lewy body dementia and/or fronto-temporal lobe dementia.

8. The method of any of claims 1 to 7, wherein the sample is selected from the group consisting of serum, plasma, and whole blood, especially plasma.

9. The method of any of claims 1 to 8, wherein the marker molecules are measured at the protein level.

10. The method of any of claims 1 to 9, wherein the individual is a human.

11. Use of A$\beta40$, A$\beta42$ and tTau as marker combination for the identification of an individual having or being at risk of developing an amyloid-positive dementia, wherein the detection of an increased combined value of the marker combination in a sample obtained from the individual as compared to the combined value as established in a reference population (control value) is indicative of the amyloid-positive dementia, wherein the sample is a blood sample.

12. The use of claim 11, wherein the use is further defined as specified in any of claims 2 to 10.


**Patentansprüche**

1. Verfahren zum Identifizieren eines Individuums mit oder mit der Gefahr des Auftretens einer Amyloid-positiven Demenz, wobei das Verfahren Folgendes umfasst

   a) Messen der Menge oder Konzentration der Markermoleküle Amyloid β40 (Aβ40), Amyloid β42 (Aβ42) und Gesamt-Tau (tTau) in einer Probe, die von dem Individuum erhalten wurde, und
   b) Identifizieren eines Individuums mit oder mit der Gefahr des Auftretens einer Amyloid-positiven Demenz durch Vergleichen des kombinierten Wertes für die in Schritt (a) bestimmten Marker mit einem Kontrollwert, wobei ein erhöhter kombinierter Wert in Bezug auf den Kontrollwert die Amyloid-positive Demenz angibt,

   wobei die Probe eine Blutprobe ist.

2. Verfahren nach Anspruch 1, wobei der kombinierte Wert durch gewichtete Berechnung der Menge oder Konzentration der Markermoleküle in den Proben erhalten wird.

3. Verfahren nach Anspruch 2, wobei der kombinierte Wert v durch gewichtete Berechnung auf der Grundlage des Gehalts (Menge oder Konzentration) von Aβ40 ([Aβ40]), des Gehalts (Menge oder Konzentration) von Aβ42 ([Aβ42]) und des Gehalts (Menge oder Konzentration) von tTau ([tTau]) gemäß der folgenden Gleichung erhalten wird:

$$v = a * [A\beta40] + b * [A\beta42] + c * [tTau] + d$$

   wobei a, b und c die Wichtungsfaktoren darstellen und d den Achsenabschnitt darstellt.

4. Verfahren nach einem der Ansprüche 1 bis 3, wobei das Individuum mit oder mit der Gefahr des Auftretens einer Amyloid-positiven Demenz identifiziert wird, wenn der kombinierte Wert für die Marker über einem Grenzwert liegt.

5. Verfahren nach Anspruch 2 oder 3, wobei die Wichtungsfaktoren durch Analysieren einer oder mehrerer Referenzpopulation(en) erhalten wurden.

6. Verfahren nach einem der Ansprüche 1 bis 5, wobei der Kontrollwert aus einer Amyloidnegativen Kontrollpopulation oder einer Amyloid-positiven Kontrollpopulation oder einer Kombination davon erhalten wurde.

7. Verfahren nach einem der Ansprüche 1 bis 6, wobei das Individuum kognitiv normal (CN) ist, einen subjektiven kognitiven Verfall (SCD) aufweist, eine milde kognitive Beeinträchtigung (MCI) aufweist oder Alzheimer-Krankheit (AD), gegebenenfalls AD kombiniert mit einem anderen Typ von Demenz (gemischte Demenz), insbesondere gemischt mit vaskulärer Demenz, Lewy-Körperchen-Demenz und/oder frontotemporale Demenz aufweist.

8. Verfahren nach einem der Ansprüche 1 bis 7, wobei die Probe aus der Gruppe ausgewählt ist, die aus Serum, Plasma und Vollblut, insbesondere Plasma besteht.

9. Verfahren nach einem der Ansprüche 1 bis 8, wobei die Markermoleküle auf Proteinebene gemessen werden.

10. Verfahren nach einem der Ansprüche 1 bis 9, wobei das Individuum ein Mensch ist.

11. Verwendung von Aβ40, Aβ42 und tTau als Markerkombination für die Identifizierung eines Individuums mit oder mit der Gefahr des Auftretens einer Amyloid-positiven Demenz, wobei der Nachweis eines erhöhten kombinierten Wertes der Markerkombination in einer Probe, die von dem Individuum erhalten wurde, im Vergleich zu dem kombinierten Wert, der bei einer Referenzpopulation (Kontrollwert) festgestellt wurde, die Amyloid-positive Demenz angibt, wobei die Probe eine Blutprobe ist.

12. Verwendung nach Anspruch 11, wobei die Verwendung ferner wie in einem der Ansprüche 2 bis 10 spezifiziert definiert ist.

**Revendications**

1. Procédé d'identification d'un individu comme ayant ou étant susceptible de développer une démence à liaison amyloïde positive, le procédé comprenant

   a) la mesure dans un échantillon obtenu auprès de l'individu de la quantité ou de la concentration des molécules marqueurs amyloïde β40 (Aβ40), amyloïde β42 (Aβ42) et Tau totale (tTau), et
   b) l'identification d'un individu comme ayant ou étant susceptible de développer une démence à liaison amyloïde positive en comparant la valeur combinée pour les marqueurs déterminés à l'étape (a) à une valeur de contrôle, une valeur combinée accrue par rapport à la valeur de contrôle indiquant la démence à liaison amyloïde positive,

   dans lequel l'échantillon est un échantillon de sang.

2. Procédé selon la revendication 1, dans lequel la valeur combinée est obtenue par calcul pondéré de la quantité ou de la concentration des molécules marqueurs dans les échantillons.

3. Procédé selon la revendication 2, dans lequel la valeur combinée v est obtenue par calcul pondéré sur la base du taux (quantité ou concentration) d'Aβ40 ([Aβ40]), du taux (quantité ou concentration) d'Aβ42 ([Aβ42]) et du taux (quantité ou concentration) de tTau ([tTau]) selon l'équation :

$$v = a * [A\beta40] + b * [A\beta42] + c * [tTau] + d$$

   dans lequel a, b et c représentent les facteurs de pondération, et d représente l'ordonnée à l'origine.

4. Procédé selon l'une quelconque des revendications 1 à 3, dans lequel l'individu est identifié comme ayant ou étant susceptible de développer une démence à liaison amyloïde positive si la valeur combinée des marqueurs est supérieure à une valeur seuil.

5. Procédé selon la revendication 2 ou 3, dans lequel les facteurs de pondération ont été obtenus en analysant une ou plusieurs population(s) de référence.

6. Procédé selon l'une quelconque des revendications 1 à 5, dans lequel la valeur de contrôle a été obtenue auprès d'une population de contrôle à liaison amyloïde négative ou d'une population de contrôle à liaison amyloïde positive ou d'une combinaison de celles-ci.

7. Procédé selon l'une quelconque des revendications 1 à 6, dans lequel l'individu est cognitivement normal (CN), a un déclin cognitif subjectif (DCS), a une déficience cognitive légère (DCL) ou a une maladie d'Alzheimer (MA), éventuellement une MA associée à un autre type de démence (démence mixte), en particulier mélangée à une démence vasculaire, une démence à corps de Lewy et/ou une démence fronto-temporale.

8. Procédé selon l'une quelconque des revendications 1 à 7, dans lequel l'échantillon est choisi dans le groupe constitué par le sérum, le plasma et le sang total, spécialement le plasma.

9. Procédé selon l'une quelconque des revendications 1 à 8, dans lequel les molécules marqueurs sont mesurées au niveau protéinique.

10. Procédé selon l'une quelconque des revendications 1 à 9, dans lequel l'individu est un humain.

11. Utilisation d'Aβ40, d'Aβ42 et de tTau en tant que combinaison de marqueurs pour l'identification d'un individu ayant ou étant susceptible de développer une démence à liaison amyloïde positive, dans laquelle la détection d'une valeur combinée accrue de la combinaison de marqueurs dans un échantillon obtenu auprès de l'individu par comparaison avec la valeur combinée telle qu'établie dans une population de référence (valeur de contrôle) indique la démence à liaison amyloïde positive, dans laquelle l'échantillon est un échantillon de sang.

12. Utilisation selon la revendication 11, dans laquelle l'utilisation est en outre définie comme spécifié dans l'une quelconque des revendications 2 à 10.

Fig. 1

All data

**Fig. 2**

Excluding other dementias

**Fig. 3**

**Only cog. normal and MCI**

Legend:
·—·—·— Ab42/Ab40 + Tau Model    ———— Ab42 + Ab40 + Tau Model
·········· Ab42/Ab40 Ratio    – – – – Tau/Ab42 Ratio

Fig. 4

**Cognitive normal, MCI and mild AD**

Legend:
- · — · — · —  Ab42/Ab40 + Tau Model
- ·········  Ab42/Ab40 Ratio
- ————  Ab42 + Ab40 + Tau Model
- – – – – –  Tau/Ab42 Ratio

Axis labels: True positive fraction (y-axis), False positive fraction (x-axis)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5283173 A
- US 5468614 A

**Non-patent literature cited in the description**

- **NAKAMURA et al.** *Nature*, 2018, vol. 554, 249-254
- **OKEREKE et al.** *J Alzheimers Dis*, 2009, vol. 16 (2), 277-285
- **SPIES et al.** *Curr Alzheimer Res*, 2010, vol. 7 (5), 470-476
- **JANELIDZE et al.** *Scientific Reports*, 2016, vol. 6
- **HAMPEL et al.** *Exp Gerontol*, 2010, vol. 45, 30-44
- **JACK et al.** *Alzheimer's & Dementia*, 2011, vol. 7, 257-262
- **TIJSSEN, P.** Practice and theory of enzyme immunoassays. Elsevier Science Publishers B.V., 1990, 43-78
- Immunoassay. Academic Press, 1996
- **SCOUTEN, W. H.** *Methods in Enzymology*, 1987, vol. 135, 30-65
- **FLORKOWSKI CM.** *Clin Biochem Rev*, 2008, vol. 29 (1), S83-S87
- **FAWCETT T.** *Pattern Recognition Letters*, 2006, vol. 27, 861-874